(19) Europäisches Patentamt
European Patent Office
Office européen des brevets

(11) Publication number: **0 421 587 A2**

# (12) EUROPEAN PATENT APPLICATION

(21) Application number: 90308800.3

(51) Int. Cl.5: A61K 31/52

(22) Date of filing: 09.08.90

(30) Priority: 10.08.89 GB 8918297

(43) Date of publication of application: 10.04.91 Bulletin 91/15

(84) Designated Contracting States: GR

(71) Applicant: Beecham Group p.l.c.
SB House Great West Road
Brentford Middlesex TW8 9BD(GB)

(72) Inventor: Smith, Harry, SmithKline Beecham Pharmaceuticals
Great Burgh, Yew Tree Bottom Road
Epsom, Surrey KT18 5XQ(GB)

(74) Representative: Rutter, Keith et al
Smith Kline Beecham, Corporate Patents,
Great Burgh, Yew Tree Bottom Road
Epsom Surrey KT18 5XQ(GB)

## (54) Xanthine derivatives used in asthma.

(57) A method for the treatment and/or prophylaxis of disorders associated with increased number of eosinophils and allergic disorders associated with atopy which method comprises the administration of an effective, non-toxic amount of a compound formula (I), as defined herein, to a human or non-human mammal in need of such treatment.

EP 0 421 587 A2

## NOVEL TREATMENT

This invention relates to a method for the treatment and/or prophylaxis of disorders associated with increased numbers of eosinophils and allergic disorders associated with atopy and to pharmaceutical compositions for use in such a method.

British patent specification No.1441562 discloses compounds of formula (A):

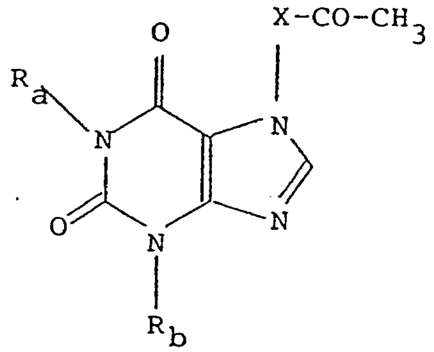

(A)

wherein $R_a$ and $R_b$ which may be the same or different, each represents a straight-chain or branched-chain alkylradical of 2 to 6 carbon atoms, or a cyclohexyl, alkoxyalkyl or hydroxyalkyl radical, and X represents a hydrocarbon radical having up to 4 carbon atoms which may be substituted by a methyl group. The compounds of the formula (A) are described as effective in increasing the blood flow through skeletal muscles while at the same time showing low toxicity.

International application, publication number WO 89/05145 (date of priority application 11th December 1987, publication date 15th June 1989) discloses that compounds of formula (A) are effective at inhibiting interleukin-1 activity, tumor necrosis factor or the activity of other leucocyte derived cytokines.

EP-0005015-A discloses that the compound of formula (A) wherein $R_a$ and $R_b$ are both n-butyl groups and X is a $CH_2$ group, i.e. 1,3-di-n-butyl-7-oxopropyl xanthine, is effective in increasing oxygen tension and contractility in ischaemic skeletal muscle and is therefore of potential use in the treatment of peripheral vascular disease.

EP-0267676 discloses that 1,3-di-n-butyl-7-(2-oxypropyl)xanthine is useful for the treatment of cerebral vascular and neuronal degenerative disorders and/or peripheral vascular disease and/or proliferate skin disease.

EP-0018135-A discloses compounds of formula (B):

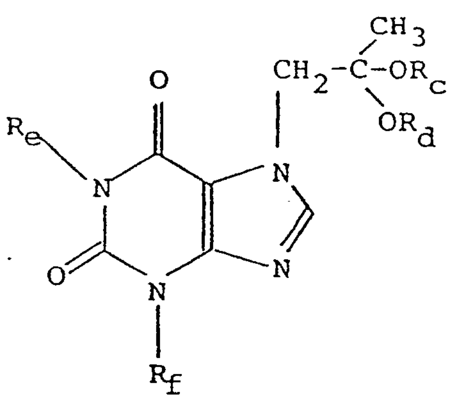

(B)

wherein

$R_c$ is a lower alkyl group and $R_d$ is a lower alkyl group; or

$R_c$ is linked to $R_d$ so that the $OR_c$ and $OR_d$ moieties and the carbon atom to which they are attached form a 1,3-dioxacyclohexa -2,2-diyl, 1,3-dioxacyclopenta-2,2-diyl or 1,3-dioxacyclohepta-2,2-diyl diradical; and

$R_e$ and $R_f$ are the same or different and are each a lower alkyl group; in which the term "lower" means containing 1 to 4 carbon atoms.

The compounds of the formula (B) are described for use in the treatment of vascular disorders such as intermittent claudication.

EP-0018136-A discloses compounds of formula (C)

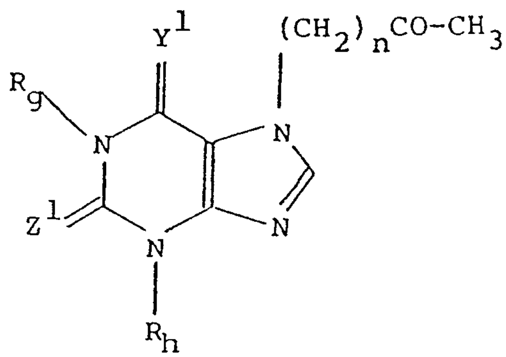

(C)

wherein

$Z^1$ is sulphur and $Y^1$ is oxygen or sulphur;

$R_g$ is an alkyl group of up to 6 carbon atoms;

$R_h$ is an alkyl group of up to 6 carbon atoms; and

n is 1; or

$Z^1$ is oxygen and $Y^1$ is sulphur;

one of $R_g$ and $R_h$ is an alkyl group of up to 6 carbon atoms and the other is an alkyl group of 2 to 6 carbon atoms; and

n is 1 or 2.

Compounds of formula (C) are disclosed as effective in improving the metabolic status of ischaemic skeletal muscle by increasing oxygen tension and/or contractility in the tissue, and are thus of potential use as agents for the treatment of peripheral vascular disease such as intermittent claudication.

EP-0042706-A discloses compounds of formula (D):

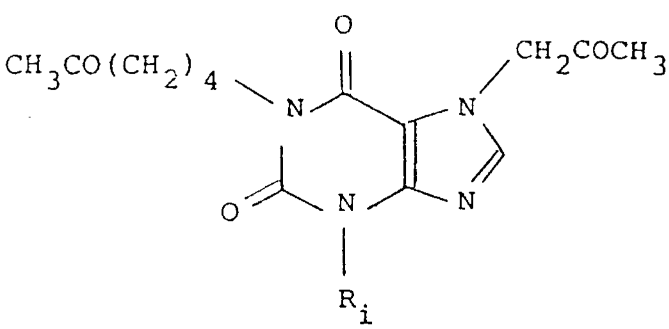

(D)

wherein $R_i$ is an alkyl group of 1 to 4 carbon atoms. The compounds of formula (D) are described as active in increasing oxygen tension in ischaemic skeletal muscle and as useful in the treatment of peripheral vascular disorders such as intermittent claudication.

It has now surprisingly been discovered that compounds of formulae (A), (B), (C), (D) and 1,3-di-n-butyl-7-(2-oxypropyl)xanthine are indicated to be good inhibitors of induced blood eosinophilia and they are therefore potentially useful in the treatment and/or prophylaxis of disorders associated with increased numbers of eosinophils, such as asthma, and allergic disorders associated with atopy, such as urticaria, eczema and rhinitis.

Accordingly, the present invention provides a method for the treatment and/or prophylaxis of disorders associated with increased numbers of eosinophils, such as asthma, and allergic disorders associated with atopy, such as urticaria, eczema and rhinitis, which method comprises the administration of an effective, non-toxic amount of a compound of formula (I):

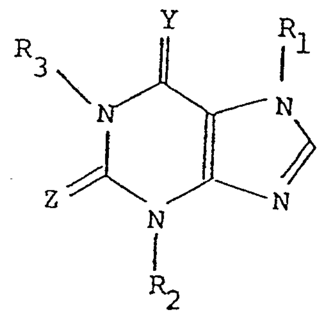

(I)

wherein

(i) $R_1$ is a group $-X-T-CH_3$ where X is a hydrocarbon radical having up to 4 carbon atoms which may be substituted by a methyl group and T is -CO- or -C(OH)-;

$R_2$ and $R_3$, which may be the same or different, each represents a straight-chain or branched-chain alkyl radical of 1 to 6 carbon atoms, or a cyclohexyl, alkoxyalkyl or hydroxyalkyl radical, and

Y and Z each represent oxygen;

(ii) $R_1$ is a group $-CH_2C(OR_4)(OR_5)CH_3$ wherein $R_4$ is an alkyl radical of 1 to 4 carbon atoms and $R_5$ is an alkyl radical of 1 to 4 carbon atoms or $R_4$ is linked to $R_5$ so that the $OR_4$ and $OR_5$ moieties and the carbon atoms to which they are attached form a 1,3-dioxacylohexa-2,2-diyl, 1,3-dioxacyclopenta-2,2-diyl or 1,3-dioxacyclohepta-2,2-diyl diradical,

$R_2$ and $R_3$ are the same or different and are each an alkyl radical of 2 to 6 carbon atoms, and Y and Z each represent oxygen;

(iii) $R_1$ is a group $-CH_2COCH_3$,

$R_2$ is an alkyl radical of 1 to 6 carbon atoms,

$R_3$ is an alkyl radical of 1 to 6 carbon atoms,

Z is sulphur and Y is oxygen or sulphur;

(iv) $R_1$ is a group $-(CH_2)_nCOCH_3$ where n is 1 or 2,

one of $R_2$ and $R_3$ is an alkyl radical of 1 to 6 carbon atoms and the other is an alkyl radical of 2 to 6 carbon atoms,

Z is oxygen and Y is sulphur; or

(v) $R_1$ is a group $-CH_2COCH_3$,

$R_2$ is an alkyl radical of 1 to 4 carbon atoms,

$R_3$ is a group $CH_3CO(CH_2)_4-$, and

Y and Z each represent oxygen.

In a further aspect, the present invention provides the use of a compound of formula (I) for the manufacture of a medicament for the treatment and/or prophylaxis of disorders associated with increased numbers of eosinophils, such as asthma, and allergic disorders associated with atopy, such as urticaria, eczema and rhinitis.

The present invention also provides a pharmaceutical composition for the treatment and/or prophylaxis of disorders associated with increased number of eosinophils, such as asthma, and allergic disorders associated with atopy, such as urticaria, eczema and rhinitis, comprising a compound of formula (I) and a pharmaceutically acceptable carrier therefor.

Suitably, for the compounds of formula (I) $R_1$ is a group $-X-CO-CH_3$ where X is a hydrocarbon radical having up to 4 carbon atoms which may be substituted by a methyl group,

$R_2$ and $R_3$, which may be the same or different, each represents a straight-chain or branched-chain alkyl radical of 2 to 6 carbon atoms, or a cyclohexyl, alkoxyalkyl or hydroxyalkyl radical, and Y and Z each represent oxygen.

Suitably, $R_2$ is n-butyl. Suitably, $R_3$ is n-butyl. Favourably, $R_2$ and $R_3$ each represent n-butyl. Suitably, $R_1$ is $CH_2COCH_3$.

Examples of compounds of formula (I) include: 1,3-di-n-butyl-7-(2-oxopropyl)xanthine; 1,3-di-n-butyl-7-(2-hydroxypropyl)xanthine; 1-n-butyl-3-ethyl-7-(2-oxopropyl)xanthine; 3-isobutyl-1-methyl-7-(2-oxopropyl)-xanthine; 1,3-di-n-butyl-7-(2-oxopropyl)-2-thioxanthine; or 3-n-butyl-1-(5-oxohexyl)-7-(2-oxopropyl)xanthine. Preferably, the compound of formula (I) is 1,3-di-n-butyl-7-(2-oxopropyl)xanthine.

The compounds of formula (I) may be prepared according to the appropriate procedures described GB

1441562, EP 0267676A, EP 0018135A, EP 0018136A or EP 0042706A.

Preferably, pharmaceutical compositions are in unit dosage form and in a form adapted for use in the medical or veterinarial fields. Such preparations may conveniently be in a pack form accompanied by written or printed instructions in accordance with the invention. A unit dose will normally contain 0.01 to 500 mg of the compound of formula (I), more suitably 1 to 500 mg, such as 1 to 100 mg or 2 to 50 mg and, especially for inhaled administration, 0.01 to 10 mg such as 0.01 to 5 mg or 2 to 5 mg.

The drug may be formulated for administration by any suitable route, the preferred route depending upon the disorder for which treatment is required, and is preferably in unit dosage form or in a form that a human patient may administer to himself in a single dosage. Advantageously, the composition is suitable for oral, rectal, topical, parenteral, intravenous or intramuscular administration or through the respiratory tract. Preparations may be designed to give slow release of the active ingredient.

Compositions may, for example, be in the form of tablets, capsules, sachets, vials, powders, granules, lozenges, reconstitutable powders, or liquid preparations, for example solutions or suspensions, or suppositories. Compositions which are especially suitable for administration to the respiratory tract and for topical administration are discussed in more detail below.

The compositions, for example those suitable for oral administration, may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable setting agents such as sodium lauryl sulphate.

Solid compositions may be obtained by conventional methods of blending, filling tabletting or the like. Repeated blending operations may be used to distribute the active agent throughout those compositions employing large quantities of fillers. When the composition is in the form of a tablet, powder, or lozenge, any carrier suitable for formulating solid pharmaceutical compositions may be used, examples being magnesium stearate, starch, glucose, lactose, sucrose, rice flour and chalk. Tablets may be coated according to methods well known in normal pharmaceutical practice, in particular with an enteric coating. The composition may also be in the form of an ingestible capsule, for example of gelatin containing the compound, if desired with a carrier or other excipients.

Compositions for oral administration as liquids may be in the form of, for example, emulsions, syrups, or elixirs, or may be presented as a dry product for reconstitution with water or other suitable vehicle before use. Such liquid compositions may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; aqueous or non-aqueous vehicles, which include edible oils, for example almond oil, fractionated coconut oil, oily esters, for example esters of glycerine, or propylene glycol, or ethyl alcohol, glycerine, water or normal saline; preservatives, for example methyl or propyl p-hydroxybenzoate or sorbic acid; and if desired conventional flavouring or colouring agents.

The drug may also be administered by a non-oral route. In accordance with routine pharmaceutical procedure, the compositions may be formulated, for example for rectal administration as a suppository or for presentation in an injectable form in an aqueous or non-aqueous solution, suspension or emulsion in a pharmaceutically acceptable liquid, e.g. sterile pyrogen-free water or a parenterally acceptable oil or a mixture of liquids, which may contain bacteriostatic agents, anti-oxidants or other preservatives, buffers or solutes to render the solution isotonic with the blood, thickening agents, suspending agents or other pharmaceutically acceptable additives. Such forms will be presented in unit dose form such as ampoules or disposable injection devices or in multi- dose forms such as a bottle from which the appropriate dose may be withdrawn or a solid form or concentrate which can be used to prepare an injectable formulation.

Compositions may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, such as from 0.1 to 50 microns, preferably less than 10 microns, for example from 1 to 10 microns, 1 to 5 microns or from 2 to 5 microns. Where appropriate, small amounts of other anti-asthmatics and bronchodilators, for example sympathomimetic amines such as isoprenaline, isoetharine, salbutamol, phenylephrine and ephedrine; corticosteroids such as prednisolone and adrenal stimulants such as ACTH may be included.

Suitably for topical administration, the compositions may be presented as a spray or aerosol.

An amount effective to treat the disorders hereinbefore described depends on the usual factors such as the nature and severity of the disorders being treated and the weight of the mammal. Unit doses will

normally be administered once or more than once a day, for example 2, 3, 4, 5 or 6 times a day, more usually 2 to 4 times a day, such that the total daily dose is normally in the range of $1.10^{-3}$ to 50 mg/kg, suitably $1.10^{-3}$ to 10mg/kg or $1.10^{-3}$ to 5 mg/kg and, especially for inhaled administration, $1.10^{-4}$ to 1 mg/kg, suitably $1.10^{-3}$ to 1 mg/kg.

The following pharmacological data illustrate the invention but do not limit it in any way.

## PHARMACOLOGICAL DATA

### Animals

Male Charles River Sprague Dawley rats weighing between 200 to 250g were used.

### Induction of blood eosinophilia and the effects of drugs .

The method used was a modification of that described by Laycock et al (Int. Arch. Appl. Immunol, (1986). 81 , 363).

Sephadex G200, particle size 40 to 120 micron, was suspended in isotonic saline at 0.5mg/ml, and stored for 48h at 4° C. 1ml of the suspension was given intravenously to rats on days 0,2 and 5. A control group received saline. The drug was given before the Sephadex on each occasion, with a contact time expected to give maximum activity at the time of the Sephadex administration. Blood was taken from the tail vein of the rats on day 7 for the determination of total and differential leucocyte counts.

A control group of at least 6 animals was included each time a compound was evaluated. The control group received Sephadex and the vehicle without drug. The results in the drug treated animals were compared with the control group. Alternatively, if the mean for the control group for any experiment was not statistically different from the mean of the sum of all of the control groups, then the treated animal results for that experiment were compared with the mean of the sum of all the control groups.

### Total and differential leucocyte counts.

20µl samples of blood, taken from the tail vein of the rats, were added to 10ml of Isoton II and, within 30min, Zaponin (3 drops) was added, to lyse the erythrocytes. Five minutes later the total cell count was determined using a Coulter Counter Model DN. Differential leucocyte counts were carried out by fixing and staining a blood smear on a microscopic slide with May-Grunwald and Giemsa stains. A minimum of 400 cells were counted on each slide.

### Results

The effect of each test compound upon Sephadex induced eosinophilia in the rat is shown in Table 1. Each test compound was given orally 30 minutes before each injection of Sephadex ($n \geq 6$) and results are expressed as a percentage of the parallel, untreated control group.

### Toxicology

No adverse toxicological effects were indicated in the abovementioned experiments.

Table 1

| Test Compound | Dose (mg/kg p.o.) | Blood Eosinophilia expressed as the % of control, mean ± SEM |
|---|---|---|
| 1,3-di-n-butyl-7-(2-oxopropyl)xanthine | 1.50<br>6.25<br>25 | 88.9 ± 8.4<br>63.9 ± 9.1<br>55.7 ± 7.5 |
| 1,3-di-n-butyl-7-(2-hydroxypropyl)xanthine | 25 | 59 ± 8 |
| 1-n-butyl-3-ethyl-7-(2-oxopropyl)xanthine | 25 | 62 ± 13 |
| 3-isobutyl-1-methyl-7-(2-oxopropyl)xanthine | 25 | 61 ± 15 |
| 1,3-di-n-butyl-7-(2-oxopropyl)-2-thioxanthine | 25 | 61 ± 8 |
| 3-n-butyl-1-(5-oxohexyl)-7-(2-oxopropyl)xanthine | 25 | 77 ± 9 |

## Claims

1. The use of a compound of formula (I):

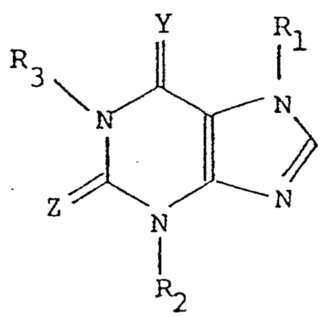

(I)

wherein:

(i) $R_1$ is a group $-X-T-CH_3$ where X is a hydrocarbon radical having up to 4 carbon atoms which may be substituted by a methyl group and T is -CO- or -C(OH)-;

$R_2$ and $R_3$, which may be the same or different, each represents a straight-chain or branched-chain alkyl radical of 1 to 6 carbon atoms, or a cyclohexyl, alkoxyalkyl or hydroxyalkyl radical, and

Y and Z each represent oxygen;

(ii) $R_1$ is a group $-CH_2C(OR_4)(OR_5)CH_3$ wherein $R_4$ is an alkyl radical of 1 to 4 carbon atoms and $R_5$ is an alkyl radical of 1 to 4 carbon atoms or $R_4$ is linked to $R_5$ so that the $OR_4$ and $OR_5$ moieties and the carbon atoms to which they are attached form a 1,3-dioxacylohexa-2,2-diyl,1,3-dioxacyclopenta-2, 2-diyl or 1,3-dioxacyclohepta-2,2-diyl diradical,

$R_2$ and $R_3$ are the same or different and are each an alkyl radical of 2 to 6 carbon atoms, and Y and Z each represent oxygen;

(iii) $R_1$ is a group $-CH_2COCH_3$,

$R_2$ is an alkyl radical of 1 to 6 carbon atoms,

$R_3$ is an alkyl radical of 1 to 6 carbon atoms,

Z is sulphur and Y is oxygen or sulphur;

(iv) $R_1$ is a group $-(CH_2)_nCOCH_3$ where n is 1 or 2,

one of $R_2$ and $R_3$ is an alkyl radical of 1 to 6 carbon atoms and the other is an alkyl radical of 2 to 6 carbon atoms,

Z is oxygen and Y is sulphur; or

(v) $R_1$ is a group $-CH_2COCH_3$,

$R_2$ is an alkyl radical of 1 to 4 carbon atoms,

$R_3$ is a group $CH_3CO(CH_2)_4-$, and

Y and Z each represent oxygen; for the manufacture of a medicament for the treatment and/or prophylaxis of disorders associated with increased numbers of eosinophils and allergic disorders associated with atopy.

2. A method according to claim 1, wherein the compound of formula (I) is selected from the group consisting of:
1,3-di-n-butyl-7-(2-oxopropyl)xanthine; 1,3-di-n-butyl-7-(2-hydroxypropyl)xanthine; 1-n-butyl-3-ethyl-7-(2-oxopropyl)xanthine; 3-isobutyl-1-methyl-7-(2-oxopropyl)xanthine; 1,3-di-n-butyl-7-(2-oxopropyl)-2-thioxanthine; and 3-n-butyl-1-(5-oxohexyl)-7-(2-oxopropyl)xanthine.

3. A method according to claim 1, wherein the compound of formula (I) is 1,3-di-n-butyl-7-(2-oxopropyl)xanthine.

4. A method according to claim 1, for the treatment and/or prophylaxis of asthma.

5. A method according to claim 1, wherein the compound of formula (I) is administered in unit dosage form.

6. A method according to claim 5, wherein the unit dosage form comprises 0.01 to 500mg of medicament.

7. A method according to claim 1, wherein the compound of formula (I) is adapted for administration to the respiratory tract.

8. A method according to claim 7, wherein the administration is via a snuff, aerosol, nebuliser or insufflation.

9. A method according to claim 8, wherein the administration is via insufflation.

10. A method according to claim 9, wherein the particles have diameters of from 1 to 10 microns.